(19) Europäisches Patentamt
European Patent Office
Office européen des brevets

(11) **EP 2 557 525 A1**

(12) **EUROPEAN PATENT APPLICATION**

(43) Date of publication:
**13.02.2013 Bulletin 2013/07**

(51) Int Cl.:
**G06K 9/00** (2006.01)  **G06T 7/00** (2006.01)

(21) Application number: **11461530.5**

(22) Date of filing: **08.08.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Instytut Biologii Doswiadczalnej IM.M. Nenckiego Pan**
**02-093 Warszawa (PL)**

(72) Inventors:
• **Ruszczycki, Blazej**
  **02-093 Warszawa (PL)**
• **Wlodarczyk, Jakub**
  **02-093 Warszawa (PL)**
• **Kaczmarek, Leszek**
  **02-093 Warszawa (PL)**

(74) Representative: **Pawlowski, Adam**
**Eupatent.PL**
**Al. Kosciuszki 23/25**
**90-418 Lodz (PL)**

(54) **A method and a system for processing an image comprising dendritic spines**

(57) A computer-implemented method for processing an image comprising dendritic spines, the method comprising the steps of obtaining the image comprising at least one dendritic spine (110), obtaining the coordinates of the tip point (311) and the base point (312), detecting the skeleton (317) of the dendritic spine (110) by analyzing the brightness of consecutive image portions (316) arranged perpendicularly to an axis extending through the tip point (311) and the base point (312) and for each image portion (316) selecting the brightest point distanced not more than a predefined threshold ($\varepsilon$) from the brightest point (314) of the previous image portion (316), detecting the contour (319) of the dendritic spine (310) by analyzing the brightness of consecutive image portions (318) arranged perpendicularly to the skeleton (317) and selecting the contour points (320) as points having brightness lower than the brightness (B) of the skeleton point multiplied by a brightness factor ($\eta$).

Fig. 1

## Description

The present invention relates to image analysis and processing related to segmenting an image comprising dendritic spines.

A dendritic spine is a membrane protrusion from a neuron's dendrite that form postsynaptic component of synapses in the brain and typically receives input from a presynaptic part located on axon. Most spines have a bulbous head and a thin neck that connects the head to the shaft of the dendrite. The dendrites of a single neuron can contain hundreds to thousands of dendritic spines. Dendritic spines have a length of about 0,2 to 2 micrometers. The spine shape and volume is thought to be correlated with the strength and maturity of each spine-synapse. It was shown that morphology of the spine can be involved in synaptic plasticity as well as in learning and memory. Thus detailed and quantitative analysis of dendritic spine morphology is appealing issue of contemporary neuroscience. Knowledge about spine morphology is important to develop new tools and adequate treatments to treat neurodegenerative disorders and may also have important diagnostic and therapuetic consequences. Moreover, the morphology of the spines is thought to be correlated with medical substances applied to the subject, therefore by analyzing the spine morphology, the substance effects can be determined.

Therefore, there is a need for image processing methods allowing efficient analysis of the shape of dendritic spines.

A PCT application WO06125188A1 presents a method for characterizing one or more neurons, comprising detecting dendritic spines utilizing a grassfire process. The method is particularly efficient for detecting separated spine heads. However, no detailed description is provided how to precisely detect the contours of the spine.

A US patent application US20020004632A1 presents a method for determining neuronal morphology and effect of substances thereon, involving detecting dendritic spines. The length for a spine fully or partially attached to its respective dendrite is determined by the distance from the center of mass corresponding to base boundary points associated with the fully or partially attached spine to a furthest spine volume element corresponding to the fully or partially attached spine.

There methods known so far are not accurate and cannot properly detect dendritic spines of unusual shapes, such as bent spines, nor are not immune to image artifacts, such as halo around the dendrite The method is especially suitable to images containing high amount of noise.

The aim of the present invention is to provide an alternative, efficient method for processing an image comprising dendritic spines to properly detect the spine shape.

The object of the invention is a computer-implemented method for processing an image comprising dendritic spines, the method comprising the steps of obtaining the image comprising at least one dendritic spine, obtaining the coordinates of the tip point and the base point, detecting the skeleton of the dendritic spine by analyzing the brightness of consecutive image portions arranged perpendicularly to an axis extending through the tip point and the base point and for each image portion selecting the brightest point distanced not more than a predefined threshold ($\varepsilon$) from the brightest point of the previous image portion, detecting the contour of the dendritic spine by analyzing the brightness of consecutive image portions arranged perpendicularly to the skeleton and selecting the contour points as points having brightness lower than the brightness (B) of the skeleton point multiplied by a brightness factor ($\eta$).

Preferably, the contour points are selected as points at which the plot of brightness of the image portion transits the point having the brightness lower than the brightness (B) of the skeleton point multiplied by a brightness factor ($\eta$) at a furthest distance from the skeleton.

Preferably, within a halo region adjacent to the base point, the contour points are selected as points having brightness lower than

$$(\eta - (L_{HALO} - L) * \eta_1) * B$$

wherein

$L_{HALO}$ is the vertical height of the halo region measured from the base point,

L is the vertical distance of the spine point belonging to the analyzed image portion from the base point,

$\eta_1$ is a halo brightness correction factor lower than the brightness factor ($\eta$).

Preferably, the image is 2-dimensional and the image portions are lines. Preferably, the detection of the spine and of the contour is limited to a triangular region having a shape of an inverted isosceles triangle with its base line along a horizontal line passing through the tip point and the other arms extending from the base point at a predefined angle. Preferably, when all points of the line arranged perpendicularly to the skeleton have a brightness higher than the brightness (B) of the skeleton point multiplied by a brightness factor ($\eta$), then the end points of the line limited by the triangular region are selected as the contour points.

Preferably, the image is 3-dimensional and the image portions are planes. Preferably, the detection of the spine and of the contour is limited to a conical region having a shape of an inverted cone with its base plane along a horizontal plane passing through the tip point and the side wall extending from the base point at a predefined angle. Preferably, all points of the plane arranged perpendicularly to the skeleton have a brightness higher than the brightness (B) of the skeleton point multiplied by a brightness factor ($\eta$), then the end points of the plane limited by the conical region are selected as the contour points.

Preferably, the method further comprises the step of approximating the set of contour points to a curve.

Preferably, the method further comprises the step of determining at least one morphological parameter of the dendritic spine, such as the length if the skeleton, the width of the head and the width of the neck, based on the determined skeleton and/or the contour of the dendritic spine.

Another object of the invention is a computer-implemented system comprising means configured to perform the steps of the method according to the invention.

The object of the invention is also a computer program comprising program code means for performing all the steps of the computer-implemented method according to the invention when said program is run on a computer.

The present invention is shown by means of exemplary embodiments on a drawing, in which:

Fig. 1 shows an exemplary image comprising dendritic spines,
Fig. 2 shows the steps of the method according to the invention
Fig. 3A shows the steps of the method for detecting the skeleton of the dendritic spine and Figs. 3B-3D show associated images and plots.
Fig. 4A shows the steps of the method for detecting the contours of the dendritic spine and Figs. 4B-4C show associated images and plots.

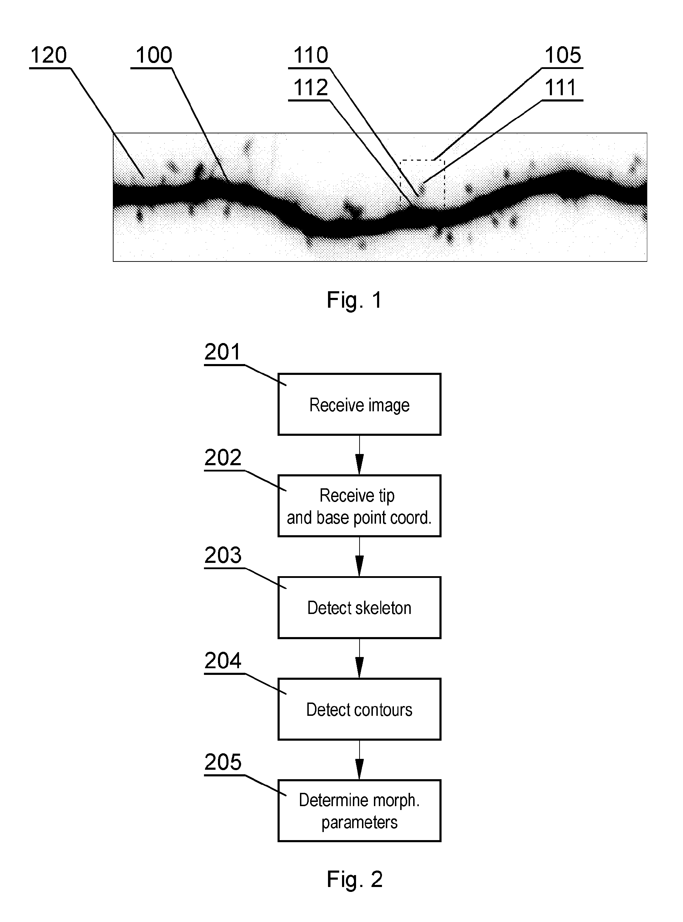

Fig. 1 shows an exemplary image comprising a dendrite 100 with dendritic spines 110, shown as an inverse image to improve the visibility. The image has been acquired by a fluorescence confocal microscope, with resulting pixel size 70nm. The presented embodiment relates to a 2-dimensional image, but it can be used with 3-dimensional images in an equivalent manner as well.

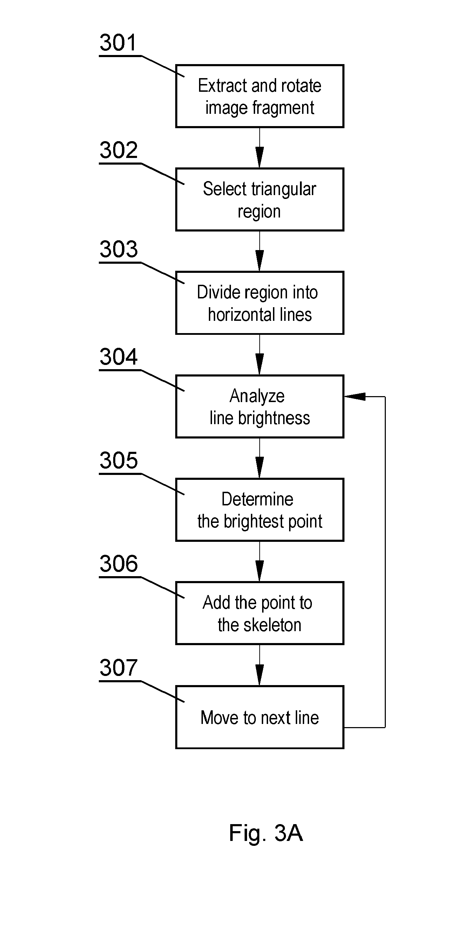

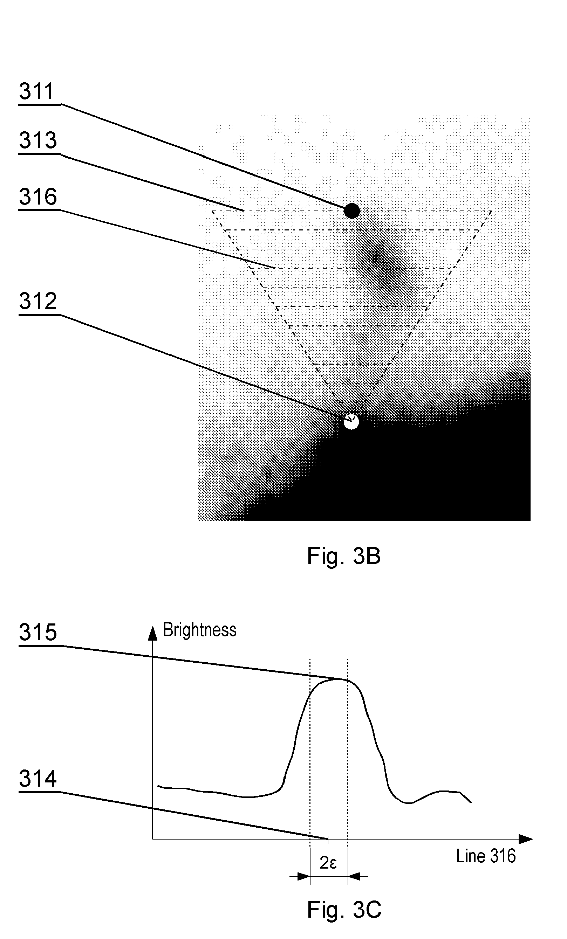

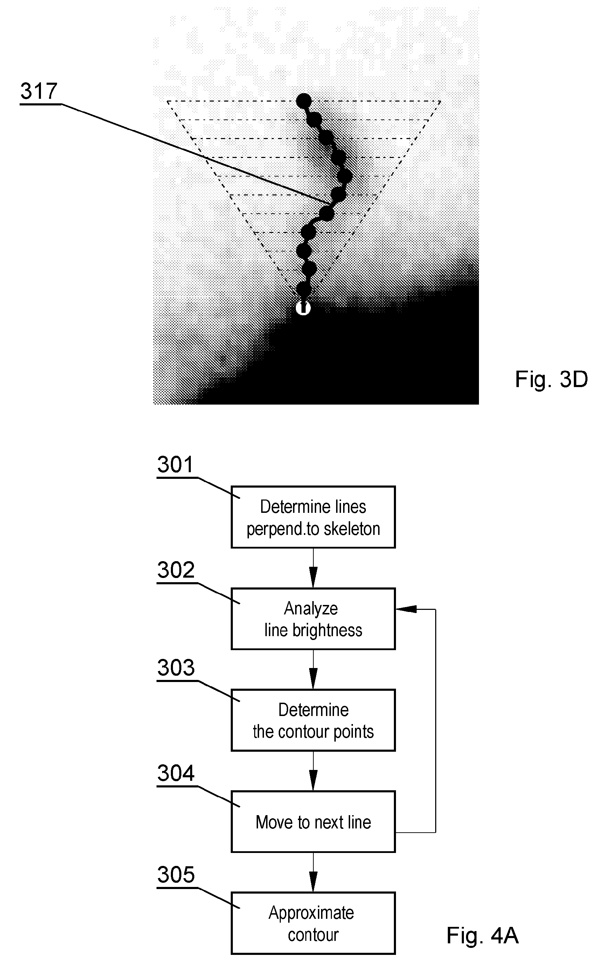

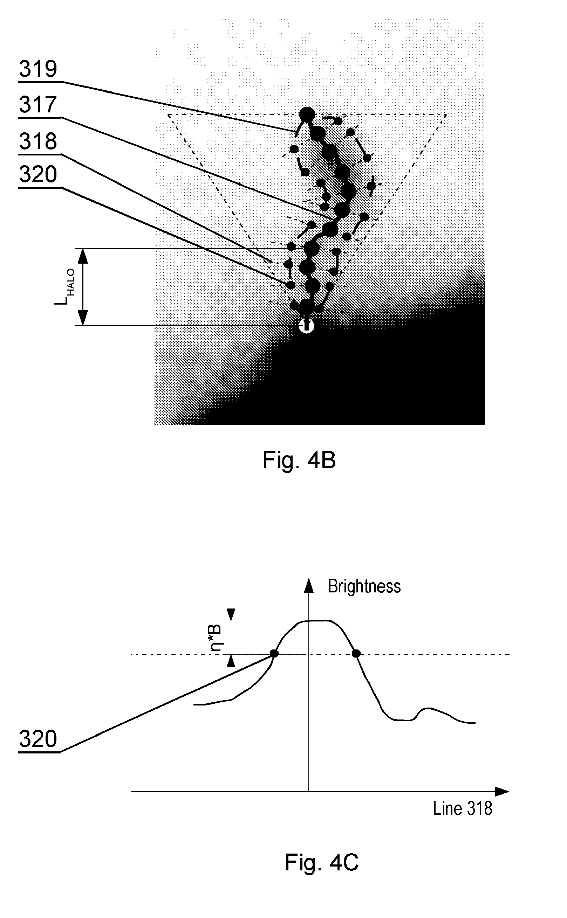

Fig. 2 shows the steps of the method according to the invention. The method starts in step 201 by receiving the image to be processed, such as the confocal microscope image shown in Fig. 1. Next, in step 202, coordinates of two points are received, namely the coordinates of the tip point 111 and the base point 112 of a dendritic spine which is to be segmented from the image, as shown in Fig. 1. The coordinates of the tip and base points may be defined by another algorithm or may be defined manually by the user. Next, in step 203, the skeleton of the dendritic spine is detected, as shown in Fig. 3. Then in step 204 the contours of the dendritic spine are detected, as shown in Fig. 4. After that, in step 205 various morphological parameters of the dendritic spine can be determined, such as the skeleton length, the size of the head, the size of the neck, the shape type (stubby, thin, mushroom) etc. After the dendritic spine is segmented from the image, the method may be reexecuted to process another dendritic spine on the image. The morphological data obtained for a plurality of dendritic spines can be used for various medical analysis applications.

Fig. 3A shows the steps of the method for detecting the skeleton of the dendritic spine 310 and Figs. 3B-3D show associated images and plots. First, in step 301 a fragment of the image comprising the tip 311 and base 312 points of the dendritic spine is extracted from the whole image and rotated such as to align the tip 311 and base 312 points along the central vertical axis. Fig. 3B shows (enlarged) the extracted and rotated fragment 105 of the image of Fig. 1. Next, in step 302, a triangular region is selected, having a shape of an inverted isosceles triangle with its base line 313 along a horizontal line passing through the tip of the dendritic spine and the other arms extending from the base point 312 of the dendritic spine at a predefined angle, such as 60 degrees. The further processing of the image is limited to the region within the triangle, such as to exclude at least part of the image comprising other dendritic spines. Next, in step 303 the triangular region is divided into horizontal image portions 316, which for the 2-dimensional image are lines, along which the image is to be processed sequentially, starting from the top line running through the tip 311. For each line, the brightness of the image is analyzed in step 304, which may be plotted as shown in example of Fig. 3C. For each line, the brightest point 315 is determined in step 305, which is within a distance $\varepsilon$ from the position 314 of previous brightest point. The distance $\varepsilon$ may be set by the user to control the accuracy of the algorithm, preferred values for the image with resolution such as shown in Fig. 1 are 2 to 5 pixels. For the first line, the previous brightest point is the tip point 311. This guarantees that all brightest points determined for consecutive lines will belong to the same dendritic spine. The determined brightest point is designated as forming a part of the skeleton in step 306 and the procedure moves to the next line in step 307. After all lines are processed, the skeleton of the dendritic spine is defined by a number of points and a curve 317 passing through these points as shown in Fig. 3D.

Fig. 4A shows the steps of the method for detecting the contours of the dendritic spine and Figs. 4B-4D show associated images and plots. First, in step 401, a plurality of image portions 318, which for the 2-dimensional image are lines, and which are perpendicular to the skeleton 317 of the dendritic cell 310 are determined, as shown in Fig. 4B. Next, for each line 318, in step 402 the image brightness along the line 318 is analyzed as shown in Fig. 4C. Moreover, as shown in Fig. 1, the image of the dendritic spine may comprise a halo 120 close to the dendrite. For such images, the halo region H can be determined by analyzing the minimum brightness of the image across the horizontal lines and determining the start of the halo region where the average brightness exceeds for example 20 % of the highest brightness. In step 403 the contour points 320 are selected, which are points at opposite sides of the line centre, i.e. the point of the skeleton. In the area outside the halo region, the contour points 320 are selected as points which have a brightness lower than η*the brightness B of the central point. Along the line 318 there could be more than one transition through a threshold set by η*the brightness B of the central point due to the artifacts such as dark spots inside the spine or in the images in which the pixels on the spine surface are the brightest (using e.g. lipofilic fluorescent dye (DiI) to visualize the spines). In this case the furthest transition from the central point within the interval W set by the user (which represents maximum allowable width of the spine) is used as the position of the spine contour along the line 318. If no such transition is found and the brightness of all pixels along the line 318 is larger than η*the brightness B of the central point, the line 318 is classified as being located inside the dendrite and is used to terminate the spine contour. The coefficient η can be configured by the user to determine the accuracy of the method and adjust it to the quality of the image. For good quality images with a high contrast, the coefficient η can be set to approximately 80%, as shown in Fig. 4C. In case the skeleton points belongs to the area of the halo region, the contour points 320 are classified as points which have a brightness lower than:

$$(\eta - (L_{HALO}-L) * \eta_1) * B$$

wherein

$L_{HALO}$ is the height of the halo region measured across the vertical axis

L is the height of the spine point belonging to the analyzed line 318

$\eta_1$ is a halo brightness correction factor, set to a value lower than η, e.g. to 30%

After the contour points 320 for the line are detected, the procedure moves to the next line in step 404. Next, the contour 319 is approximated to a curve in step 405 by known curve approximation algorithms.

The method presented above for the 2-dimensional image can be used for 3-dimensional images in an equivalent manner, wherein image portions 316, 318 are not lines, but planes. Furthermore, the analysis can be limited to a conical region having a shape of an inverted cone with its base plane 313 along a horizontal plane passing through the tip point 311 and the side wall extending from the base point 312 at a predefined angle.

The aforementioned method may be performed and/or controlled by one or more computer programs run in a computer system. Such computer programs are typically executed by utilizing the computing resources of a processing unit which can be embedded within various signal processing units, such as personal computers or dedicated microscope controllers.

## Claims

1. A computer-implemented method for processing an image comprising dendritic spines, the method comprising the steps of:

    - obtaining the image comprising at least one dendritic spine (110),
    - obtaining the coordinates of the tip point (311) and the base point (312)
    - detecting the skeleton (317) of the dendritic spine (110) by analyzing the brightness of consecutive image portions (316) arranged perpendicularly to an axis extending through the tip point (311) and the base point (312) and for each image portion (316) selecting the brightest point distanced not more than a predefined threshold ($\varepsilon$) from the brightest point (314) of the previous image portion (316),
    - detecting the contour (319) of the dendritic spine (310) by analyzing the brightness of consecutive image portions (318) arranged perpendicularly to the skeleton (317) and selecting the contour points (320) as points having brightness lower than the brightness (B) of the skeleton point multiplied by a brightness factor (η).

2. The method according to claim 1, wherein the contour points (320) are selected as points at which the plot of brightness of the image portion (318) transits the point having the brightness lower than the brightness (B) of the skeleton point multiplied by a brightness factor (η) at a furthest distance from the skeleton (317).

3. The method according to claim 1 or 2, wherein within a halo region adjacent to the base point (312), the contour points (320) are selected as points having brightness lower than:

$$(\eta - (L_{HALO}-L) * \eta_1) * B$$

wherein

$L_{HALO}$ is the vertical height of the halo region measured from the base point (312),

L is the vertical distance of the spine point belonging to the analyzed image portion (318) from the base point (312),

$\eta_1$ is a halo brightness correction factor lower than the brightness factor (η).

4. The method according to any of previous claims, wherein the image is 2-dimensional and the image portions (316, 318) are lines.

5. The method according to claim 4, wherein the detection of the spine (317) and of the contour (319) is limited to a triangular region having a shape of an inverted isosceles triangle with its base line (313) along a horizontal line passing through the tip point (311) and the other arms extending from the base point (312) at a predefined angle.

6. The method according to claim 5, wherein when all points of the line (318) arranged perpendicularly to the skeleton (317) have a brightness higher than the brightness (B) of the skeleton point multiplied by a brightness factor ($\eta$), then the end points of the line (318) limited by the triangular region are selected as the contour points (320).

7. The method according to any of previous claims, wherein the image is 3-dimensional and the image portions (316, 318) are planes.

8. The method according to claim 7, wherein the detection of the spine (317) and of the contour (319) is limited to a conical region having a shape of an inverted cone with its base plane (313) along a horizontal plane passing through the tip point (311) and the side wall extending from the base point (312) at a predefined angle.

9. The method according to claim 7, wherein when all points of the plane (318) arranged perpendicularly to the skeleton (317) have a brightness higher than the brightness (B) of the skeleton point multiplied by a brightness factor ($\eta$), then the end points of the plane (318) limited by the conical region are selected as the contour points (320).

10. The method according to any of previous claims, further comprising the step of approximating the set of contour points (320) to a curve (319).

11. The method according to any of previous claims, further comprising the step of determining at least one morphological parameter of the dendritic spine, such as the length if the skeleton, the width of the head and the width of the neck, based on the determined skeleton (317) and/or the contour (319) of the dendritic spine (310).

12. A computer-implemented system comprising means configured to perform the steps of the method according to claims 1-11.

13. A computer program comprising program code means for performing all the steps of the computer-implemented method according to any of claims 1-11 when said program is run on a computer.

Fig. 1

Fig. 2

301 — Extract and rotate image fragment

302 — Select triangular region

303 — Divide region into horizontal lines

304 — Analyze line brightness

305 — Determine the brightest point

306 — Add the point to the skeleton

307 — Move to next line

Fig. 3A

311

313

316

312

Fig. 3B

315

Brightness

314

Line 316

2ε

Fig. 3C

317

Fig. 3D

301 — Determine lines
perpend.to skeleton

302 — Analyze
line brightness

303 — Determine
the contour points

304 — Move to next line

305 — Approximate
contour

Fig. 4A

319

317

318

320

$L_{HALO}$

## Fig. 4B

Brightness

$\eta*B$

320

Line 318

## Fig. 4C

Europäisches
Patentamt

European
Patent Office

Office européen
des brevets

# EUROPEAN SEARCH REPORT

Application Number

EP 11 46 1530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FIGUEIREDO M A T ET AL: "A NONSMOOTHING APPROACH TO THE ESTIMATION OF VESSEL CONTOURS IN ANGIOGRAMS", IEEE TRANSACTIONS ON MEDICAL IMAGING, IEEE SERVICE CENTER, PISCATAWAY, NJ, US, vol. 14, no. 1, 1 March 1995 (1995-03-01), pages 162-172, XP000507934, ISSN: 0278-0062, DOI: 10.1109/42.370413 * the whole document * | 1,2,4,7, 10-13 | INV. G06K9/00 G06T7/00 |
| A | SMETS C ET AL: "A KNOWLEDGE-BASED SYSTEM FOR THE DELINEATION OF BLOOD VESSELS ON SUBTRACTION ANGIOGRAMS", PATTERN RECOGNITION LETTERS, ELSEVIER, AMSTERDAM, NL, vol. 8, no. 2, 1 September 1988 (1988-09-01), pages 113-121, XP000004984, ISSN: 0167-8655, DOI: 10.1016/0167-8655(88)90052-9 * the whole document * | 1,2,4,7, 10-13 | |
| A | MILES F P ET AL: "Matched filter estimation of serial blood vessel diameters from video images", IEEE TRANSACTIONS ON MEDICAL IMAGING, IEEE SERVICE CENTER, PISCATAWAY, NJ, US, vol. 12, no. 2, 1 June 1993 (1993-06-01), pages 147-152, XP002352277, ISSN: 0278-0062, DOI: 10.1109/42.232243 * the whole document * | 1,2,4,7, 10-13 | TECHNICAL FIELDS SEARCHED (IPC) G06K G06T |

-/--

The present search report has been drawn up for all claims

| Place of search | Date of completion of the search | Examiner |
|---|---|---|
| The Hague | 2 February 2012 | Deltorn, Jean-Marc |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 557 525 A1**

Europäisches
Patentamt

European
Patent Office

Office européen
des brevets

# EUROPEAN SEARCH REPORT

Application Number

EP 11 46 1530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KHALID A AL-KOFAHI K A ET AL: "Rapid Automated Three-Dimensional Tracing of Neurons From Confocal Image Stacks", IEEE TRANSACTIONS ON INFORMATION TECHNOLOGY IN BIOMEDICINE, IEEE SERVICE CENTER, LOS ALAMITOS, CA, US, vol. 6, no. 2, 1 June 2002 (2002-06-01), XP011074824, ISSN: 1089-7771 * the whole document * | 1-13 | |
| A | US 2009/070089 A1 (KAWATO SUGURU [JP] ET AL) 12 March 2009 (2009-03-12) * the whole document * | 1-13 | |

TECHNICAL FIELDS
SEARCHED (IPC)

The present search report has been drawn up for all claims

| Place of search | Date of completion of the search | Examiner |
|---|---|---|
| The Hague | 2 February 2012 | Deltorn, Jean-Marc |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 46 1530

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2012

| Patent document cited in search report | Publication date | Patent family member(s) | | Publication date |
|---|---|---|---|---|
| US 2009070089 A1 | 12-03-2009 | US | 2009070089 A1 | 12-03-2009 |
| | | WO | 2007069414 A1 | 21-06-2007 |

EPO FORM P0459

For more details about this annex : see Official Journal of the European Patent Office, No. 12/82

**EP 2 557 525 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 06125188A1 A
- US 20020004632 A1